(19) Europäisches Patentamt
European Patent Office
Office européen des brevets

(11) **EP 0 712 303 B2**

(12) **NEW EUROPEAN PATENT SPECIFICATION**
After opposition procedure

(45) Date of publication and mention
of the opposition decision:
**17.09.2008 Bulletin 2008/38**

(45) Mention of the grant of the patent:
**21.09.2005 Bulletin 2005/38**

(21) Application number: **94925151.6**

(22) Date of filing: **27.07.1994**

(51) Int Cl.:
*A61K 9/70* (2006.01)      *A61K 31/565* (2006.01)

(86) International application number:
**PCT/US1994/008637**

(87) International publication number:
**WO 1995/003764 (09.02.1995 Gazette 1995/07)**

(54) **SKIN PATCHES COMPRISING TESTOSTERONE AND OPTIONALLY ESTROGEN**

HAUTPFLASTER ENTHALTEND TESTOSTERON UND GEGEBENENFALLS ESTROGEN

TIMBRES TRANSDERMIQUES COMPRENANT DE LA TESTOSTERONE ET OPTIONNELLEMENT DE L'OESTROGENE

(84) Designated Contracting States:
**AT BE CH DE DK ES FR GB GR IE IT LI LU MC NL PT SE**

(30) Priority: **03.08.1993 US 99923**

(43) Date of publication of application:
**22.05.1996 Bulletin 1996/21**

(60) Divisional application:
**05010937.0 / 1 604 651**

(73) Proprietor: **Watson Laboratories, Inc.**
**Salt Lake City**
**Utah 84108 (US)**

(72) Inventors:
• **EBERT, Charles D.**
**Salt Lake City, UT 84108 (US)**
• **PATEL, Dinesh C.**
**Murray, UT 84107 (US)**
• **MAZER, Norman Alan**
**Salt Lake City, UT 84124 (US)**
• **VENKATESHWARAN, Srinivasan**
**Salt Lake City, UT 84108 (US)**

(74) Representative: **Johansen, Marianne et al**
**Albihns A/S**
**Havneholmen 29**
**Building 2, 3. floor**
**1561 Copenhagen V (DK)**

(56) References cited:
EP-A- 0 416 842          WO-A-92/18107
WO-A-93/02669          US-A- 5 023 084
US-A- 5 152 997          US-A- 5 340 586

• DRUG DEVELOPMENT AND INDUSTRIAL PHARMACY (YU et al.) 1991, 17(14); "Transdermal Dual-Controlled Delivery of Testosterone and Estradiol: (I) Impact of System Design", see pages 1883-1904.
• PSYCHOSOMATIC MEDICINE, Vol. 47, No. 4, (SHERWIN et al.) 1985; "Androgen Enhances Sexual Motivation in Females: A Prospective, Crossover Study of Sex Steroid Administration in the Surgical Menopause", see pages 339-350.
• PSYCHONEUROENDOCRINOLOGY, Vol. 10, No. 3, (SHERWIN et al.), 1985; "Sex Steroids and Affect in the Surgical Menopause: A Double-Blind, Cross-Over Study", see pages 325-335.
• AMERICAN JOURNAL OF OBSTETRICS AND GYNECOLOGY, Volume 151, Number 2, (SHERWIN et al.) 1985: "Transactions of the Fortieth Annual Meeting of the Society of Obstetricians and Gynaecologists of Canada", see pages 153-160.
• Publication : origin of episodic fluctuation of plasma testosterone in women : adrenal ovarian and TeBG contribution, pp. 403-414, 1987
• Publication : Pharmacokinetics 2nd, Ch 8, pp. 319-353, Marcel Dekker, New York, 1982
• Journal of Clinical Endocrinology and Metabolism, 28, 1968, pp. 1105-1112
• Journal of Clinical Investigation, 48, 1969, pp. 696-703

## Description

This invention is in the field of transdermal drug delivery. More particularly, it is directed to skin patches and methods for providing transdermal delivery of testosterone and optionally estrogens to women.

Estrogens are commonly used in hormone replacement therapy for the treatment of post-menopausal symptoms and osteoporosis. Many women, however, continue to experience symptoms after estrogen monotherapy. The beneficial effects of androgens on the post-menopausal woman's physical and emotional well-being are only recently being recognized. Several clinical studies have established the basis of combined estrogen-androgen therapy. In a series of double-blind, controlled studies at McGill University, it was noted that patients on estrogen-androgen combination therapy had higher daily scores of well-being, energy level, and quality of sleep than patients on estrogen replacement therapy (ERT) alone. The authors also reported a beneficial effect of estrogen-androgen therapy on sexual function, and no adverse effects on lipids.

Androgens are the hormones of choice in helping restore lost sex drive, although estrogens alone may help some women. Several reports in the literature establish the beneficial effects of androgen therapy on sexual function. This is particularly important in younger, surgically post-menopausal women, who have increased motivational aspect of sexual behavior (such as desire and arousal) when treated with parenteral androgen, either alone or in combination with an estrogen, compared to estrogen alone or placebo. Justification for the addition of androgens to ERT for the prevention of osteoporosis is also found in the literature.

While the benefits of adding an androgen to an ERT regimen are well established, the dosage forms available for androgen therapy or estrogen-androgen combination therapy leave much to be desired. In particular, none of these modalities can produce serum testosterone levels that remain within normal physiological range on a daily basis. The androgens most available for clinical use are not typically the native testosterone; synthetic androgens are commonly used (methyl testosterone, for example which is known to be hepatotoxic). Native testosterone is available as implantable pellets; however this system requires an invasive administration and does not produce a stable physiological hormonal state. Injectable testosterone esters such as testosterone propionate and the long-acting testosterone enanthate produce marked supraphysiological fluctuations in hormone levels. The non- and supraphysiological nature of the current androgen replacement products are responsible for the signs and symptoms of excess androgen effect frequently seen with their use. These include virilizing symptoms such as hirsutism, male pattern baldness, voice lowering and clitoromegaly; disturbances in ovulation and menstrual function; acne and oily skin; and breast tenderness, fluid retention, irritability and depression. These problems are also present with estrogen-androgen products currently on the market. A modality producing physiological levels of testosterone and its metabolites, with or without estrogen, would have significant therapeutic benefits over existing androgen replacement products.

Many articles and patents have suggested or reported transdermal administration of testosterone. In general, these publications have focused on providing testosterone therapy to men to treat conditions such as male hypogonadism, anemia, and male osteoporosis. Much of this literature concludes that testosterone, without some form of permeation enhancement, does not pass through non-scrotal skin at practical fluxes. Most recently, U.S. 5,152,997 describes the transdermal administration of testosterone to males through non-scrotal skin under conditions (subsaturation concentrations of testosterone in the skin patch; coadministration of skin permeation enhancers) that provide practical flux levels. This literature is virtually silent on the subject of providing testosterone or combined testosterone/estrogen replacement therapy to women transdermally.

WO 92 18107 refers to contraception and treatment of benign gynecological disorders of administering a gonadotropin releasing hormone together with an estrogenic progestogen and optionally an androgen for example transdermal route.

One may provide testosterone replacement therapy to a woman in need of such therapy by administering testosterone transdermally to the woman in an amount that provides blood levels of testosterone in the woman that correspond substantially to endogenous blood levels of testosterone in healthy young adult women.

Thus, the invention provides a skin patch for providing testosterone replacement therapy transdermally to a woman in need of such therapy, the patch comprising a laminated of:

(a) a backing layer; and
(b) a matrix layer comprising a solution of testosterone in a polymeric carrier being a pressure sensitive adhesive, the matrix layer containing a testosterone concentration sufficient for administering testosterone transdermally in an amount ranging from 50 to 500 μg/day, thereby causing blood levels of testosterone to be from 15 to 80 ng/dl.

The matrix layer may further include an amount of estrogen equivalent to 25 to 200 μg/day of 17β-estradiol, thereby resulting in an estrogenic activity level equivalent to a 17β-estradiol blood level of 25 to 200 pg/ml.

Thus, one may provide combined testosterone and estrogen replacement therapy to a woman in need of such therapy by coadministering testosterone and estrogen transdermally to the woman in respective amounts that provide blood levels of testosterone and estrogen in the woman that correspond substantially to endogenous blood levels of testosterone and estrogen in healthy young adult women.

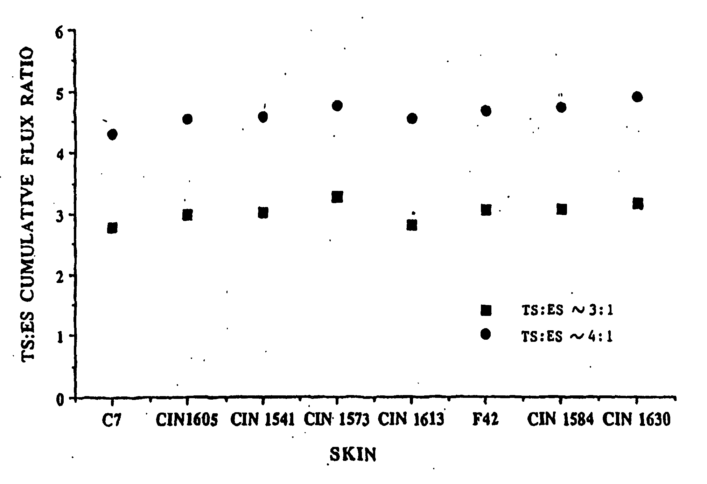

Brief Description of the Drawing

The drawing is a graph of data described in Example 2 , *infra*.

As used herein, the word "estrogen" intends 17β-estradiol; esters thereof such as estradiol-3,17-diacetate, estradiol-3-acetate, estradiol-17-acetate, estradiol-3,17-divalerate, estradiol-3-valerate, estradiol-17-valerate, and the corresponding pivalate, propionate, cypionate, benzoate, and like esters; ethinyl estradiol, estrone, and other estrogenic steroids and derivatives thereof that are suitable for transdermal administration. Correspondingly, the term "testosterone" intends testosterone and pharmaceutically acceptable derivatives thereof such as the enanthate, proprionate and cypionate esters of testosterone.

As used herein, the term "testosterone replacement therapy" intends the transdermal administration to a woman of all or a portion of the testosterone that is normally produced by a healthy-young adult woman. In this regard, in healthy adult women approximately 50% of the testosterone is produced by each of the ovaries and the adrenal glands. Thus; in most instances this therapy will be provided to women who have undergone bilateral oopherectomy, post-menopausal with diminished androgen production, and women with diminished adrenal androgen production (e.g., Addison's disease). This latter group also includes women who receive high dose corticosteroid therapy that can secondarily suppress adrenal androgen production.

As used herein the term "flux" intends the rate of transfer of hormone across skin as measured by the method of Merritt and Cooper (J Controlled Release (1984) 1:161).

The term "endogenous serum levels" as used herein intends the serum levels of testosterone or estradiol, as the case may be, that are normally found in healthy young adult women. In the case of testosterone this value is about 15 to 80 ng/dl; whereas for estradiol it is about 25 to 200 pg/mL. in the case of derivatives of testosterone or estradiol, amounts that provide equivalent androgenic or estrogenic activity, as the case may be, to the natural hormones are intended. The term "corresponds substantially" intends a permitted standard deviation of $\geq 100\%$ in the case of both testosterone and estradiol.

The term "diffusional communication" intends that when the invention patch is placed on the skin/mucosa of a woman, there is a direct or indirect diffusional pathway by which the testosterone and optionally estrogen can migrate from the hormone-containing element(s) of the patch to the skin/mucosa of the wearer.

The daily dose of testosterone that will normally be required to provide replacement therapy to females is in the range of 50 to 500 $\mu$g/day, more usually 100 to 300 $\mu$g/day. Correspondingly, the daily dose of 17β-estradiol will normally be in the range of 25 to 200 $\mu$g/day, more usually 50 to 100 $\mu$g/day. Thus, for patches in the 5 to 30 $cm^2$ diffusional area range, the flux of testosterone will be in the range of 1.7 to 100 $\mu$g/$cm^2$/day, more usually 3.3 to 60 $\mu$g/$cm^2$/day whereas the flux of estradiol will usually be in the range of 0.8 to 40 $\mu$g/$cm^2$/day, more usually 1.7 to 20 $\mu$g/$cm^2$/day.

The patches that are used to provide such daily doses of testosterone and optionally estradiol are of the matrix type. In matrix-type patches, the hormone(s) is/are homogeneously blended in a solid or semisolid polymer carrier together with other additives (e.g., permeation enhancers, plasticizers, viscosity reducing agent, and the like). The general structure and fabrication of matrix -type patches are well known in the art.

A preferred patch for administering testosterone or testosterone/estrogen in accordance with this invention is a matrix-type patch which comprises an occlusive backing that is impermeable to the testosterone/estrogen and defines the face or top surface of the patch and a solid or semisolid matrix layer comprised of a homogeneous blend of the hormone(s), a polymeric carrier, and one or more skin permeation enhancers.

The particular chemistry of the polymeric carrier is not critical. Rather, the diffusion coefficient of the hormone(s) in the carrier and the solubilities of the hormone(s) in the carrier are important. In this regard the diffusion coefficient of the hormone(s) in the carrier will usually be between $10^{-4}$ and $10^{-12}$ $cm^2$/sec, more usually $10^{-5}$ and $10^{-8}$ $cm^2$/sec. Correspondingly, the solubilities of the hormones in the carrier will usually be in the range of 1 to 200 mg/$cm^3$, more usually 10 to 100 mg/$cm^3$. The amount of testosterone and optionally estrogen in the carrier will be sufficient to provide the required daily dose of hormone(s) over the intended wearing time. The hormone(s) are preferably present in the carrier below their saturation concentrations to avoid crystallization. The patches will be typically designed to be worn for 1 to 14 days, more usually 1 to 7 days. Accordingly, the amount of testosterone in the carrier will normally be between 0.1 to 30 mg, more usually 1 to 10 mg and the amount of estrogen, when present, will usually be between 0.1 and 10 mg, more usually 0.5 and 5 mg. The thickness of the matrix layer will normally be 0.01 to 1 mm, more usually 0.025 to 0.25 mm. The area of the patch in diffusional contact with the skin will usually be between 1 and 150 $cm^2$, more usually 5 and 40 $cm^2$. The basal surface of the matrix layer may be used to affix the patch to the skin.

Examples, of specific polymers that may be used as the carrier are polyacrylates, polymethacrylates, natural and synthetic rubbers, silicone rubbers and elastomers, polyolefins, vinyl copolymers, urethanes, nylons, polyesters, polyethers, and the like.

The skin permeation enhancer(s) that are included in the matrix enhance the level of skin flux of the hormone (s). Examples, of skin permeation enhancers that may be used are those described or referred to in U.S. Pats. Nos. 5,122,383 and 5,152,997. The enhancer will usually constitute 1 to 20 wt% of the matrix, more usually 5 to 15 wt% of the matrix.

The patches of the invention may be manufactured by conventional techniques used in transdermal drug delivery device art. For instance, the hormone(s), carrier, and enhancer(s) may be mixed in the desired proportions to form a homogeneous mixture and cast or otherwise applied to a backing layer, by lamination to a release liner layer.

The following examples further illustrate the invention. Unless indicated otherwise percentages and proportions are by weight.

Examples

Example 1

Testosterone (TS) was obtained from the Upjohn Company. Sorbitan monooleate (Arlacel 80) was obtained from ICI American. Polyacrylate adhesive (Durotak 80-1196) was obtained from National Starch and Chemical Company. Release liner (polyester, S242M) was obtained from Release Technologies, Inc. and polyethylene backing film (Cotran 9720) from 3M Corporation.

The solid content of the adhesive was determined by weighing a small amount of the adhesive solution in a pre-weighed aluminum dish. The solvent was evaporated by overnight drying in a convection oven maintained at 70°C and the % solid adhesive content determined based upon the weight of the dried adhesive.

Known amounts of the adhesive solution were weighed into glass bottles. From the weight of the adhesive solution and the percent solid adhesive content, the amount of adhesive in the solution was calculated. Appropriate quantities of testosterone and sorbitan monooleate were added to the adhesive solution to yield a dried film composition in which the adhesive, testosterone, sorbitan monooleate were in a weight ratio of 83:2:15. The glass bottle was then tightly capped, sealed with parafilm, and rotated overnight until all ingredients had completely dissolved and the solution was visually clear.

About 8 ml of the adhesive/testosterone/sorbitan monooleate mixture was then dispensed on a release liner, and cast with a 250 micron gap casting knife. This cast was dried in a convention oven at 70°C for 15 minutes to yield a dry film thickness of approximately 60 microns. The backing film was then laminated onto this adhesive film using a rubber roller. This matrix laminate was used to conduct *in vitro* skin flux studies.

Similar laminates were made using other enhancers (glyceryl monooleate, methyl laurate, ascorbyl stearate, and ascorbyl palmitate) and different amounts of enhancer (5%-15% by weight), and different TS loadings at 15% enhancer loading.

The *in vitro* skin flux studies were conducted by the general method of Merritt and Cooper, supra, using modified Franz diffusion cells. Heat separated human epidermal membrane was cut into rectangular strips. The matrix was also cut into corresponding rectangular strips. After the release liner was peeled and discarded, the matrix was laminated onto the stratum corneum surface of the epidermal membrane. The skin-matrix sandwich was then cut into smaller pieces for loading onto the diffusion cells. Each piece of the skin matrix sandwich was loaded between the donor and receiver compartments of a diffusion cell, with the epidermal side facing the receiver compartment, and clamped in place. The receiver compartment was then filled with 0.02% sodium azide solution. The solubility of the testosterone in this medium is adequate to ensure sink conditions throughout the experiment. The cell was then placed in a circulating water bath calibrated to maintain the skin surface temperature at $32 \pm 1°C$.

At predetermined intervals, the entire contents of the receiver compartment was collected for TS quantitation, and the receiver compartment was refilled with fresh receptor medium, taking care to eliminate any air bubbles at the skin/solution interface. The cumulative amount of TS permeated per unit area at any time t ($Q_t$, $\mu g/cm^2$) was determined as follows:

$$Q_t = \sum_{n=0}^{t} (C_n \cdot V)/A$$

where $C_n$ is the concentration ($\mu g/ml$) of drug in the receiver sample for the corresponding sample time, V is the volume of fluid in the receiver chamber (-6.3 $cm^3$), and A is the diffusional area of the cell (0.64 $cm^2$). The slope of the best fit line to the $Q_t$ vs. t plot gives the,steady state flux ($J_{ss}$, $\mu g/cm^2/h$); the intercept of this line on the time axis gives the lag time ($t_L$, h).

Reverse-phase HPLC was used to determine the concentration of TS in samples from *in vitro*. The HPLC methodology is summarized below.

| | |
|---|---|
| Mobile Phase | 47/53 % (v/v) Acetonitrile/$H_2O$ |
| Flow Rate | 1.0 Ml/min |
| Column | Partisphere C18, 10 cm (Whatman) |
| Injection Volume | 50 $\mu$L |
| Wave Length | 245 nm |
| Retention Time | 4.5-4.8 min |
| Run Time | 6 min |

From these studies a twice-a-week (i.e., to be worn -3-4 days) prototype testosterone patch was designed. The matrix formulation of the prototype patch is Durotak 80-1196 (83%)/TS (2%)/Arlacel 80 (15%). The coating weight of the matrix is 91 $\pm$ 9.1 mg; the size is 15 cm$^2$, and the daily dose is approximately 150 $\mu$g/day.

Example 2

Estradiol (ES) was obtained from Diosynth, Inc. Other materials were obtained as described in Example 1.

Based on the studies of Example 1 two patches which coadminister TS and ES were made. One was designed to have a TS:ES flux of 4:1; the other a 3:1 flux ratio. The matrix composition of the 4:1 patch was Durotak 80-1196 (80.5%)/Arlacel 80 (15%)/ES (1.5%)/TS (3%). The matrix composition of the 3:1 patch was Durotak 80-1196 (81.5%)/ Arlacel 80 (15%)/ES (1.5%)/TS (2%). Other patch components were as in Example 1.

Flux studies were carried out on these patches as in Example 1. The drawing is a plot of the data from these studies. Specifically the drawing shows the TS:ES flux ratio for each of 8 skin samples. Based on these studies a 3:1 ratio 10-20 cm$^2$ patch targeted to deliver -150 $\mu$g/day TS and 50 $\mu$g/day ES was developed.

Example 3

A clinical study of the patches described in Examples 1 (designated TMTDS) and 2 (designated ETMTDS) (both prototypes were 15cm$^2$ in surface area) was carried out. The study was designed to evaluate the serum concentrations of testosterone and estradiol in postmenopausal women obtained by wearing the patches for 96 hr. Details of the study follow:

a) Subject Population

Twelve healthy women ranging in age from 36 to 61 years participated. All had undergone bilateral oophorectomy and hysterectomy.

b) Treatment Schedule

Each subject received treatment in a balanced randomized crossover sequence involving two treatment periods. In each treatment period one patch was worn for 96 hours. A washout period of two weeks separated the start of each period. The patches were worn on alternate sides of the upper outer quadrant of the buttocks below the waistline.

c) Blood Samples

Samples were obtained prior to, during, and following each patch application in each treatment period. Pre-application samples were used to determine the pattern of circadian variation on endogenous hormone production. They were obtained at - 24, -22, -20, -18, -16, -12, and -1 hours. During patch wearing samples were obtained at 2, 4, 6, 8, 12, 24, 36, 48, 60, 72, 84, and 96 hours. Post-administration samples were obtained at 98, 100, 108 and 120 hours. Samples were analyzed for testosterone (T), estradiol (E2) dihydrotestosterone (DHT), dihydroepiandrostenedione (DHEA), and estrone (E1) content.

d) Levels of Hormones in Serum

1. TMTDS

Serum concentrations of testosterone increased steadily after system application and reached a plateau within 8-12 hours. Even though mean (SD), uncorrected $C_{max}$ for T was 35.5 ng/dl (Range: 16-53 ng/dl), serum concentrations did not significantly differ over the dosing interval between 24 and 96 hours. Mean (SD) $C_{max}$ for estradiol and estrone were 7.5 and 27.0 pg/ml respectively. These values differ slightly from average baseline values, 3.0 for estradiol and 19.2 for estrone, and may reflect ultimate metabolic products of exogenous testosterone, but are probably not significant in quantity. DHT, the primary metabolite of testosterone, also slowly increased during the wearing period and achieved a mean (SD) $C_{max}$ of 7.8 ng/dl (Range: 4-11 ng/dl). During the dosing interval DHT concentrations remained in a fairly narrow plateau similar to T concentrations. During the TMTDS wearing period, DHEA concentrations ranged from 134-1091 ng/dl. The circadian pattern associated with DHEA concentrations was not perturbed by the exogenous delivery of testosterone from the TMTDS. Mean (SD) concentrations of each of the hormones measured during the TMTDS wearing period are tabulated in Table 1 below.

**TABLE 1**

| TIME | TESTOSTERONE (ng / dl) | | ESTRONE (pg / ml) | | ESTRADIOL (pg / ml) | | DHT (ng / dl) | | DHEA (ng / dl) | |
|---|---|---|---|---|---|---|---|---|---|---|
| Hours | MEAN | St. Dev | MEAN | St. Dev | MEAN | St. Dev | MEAN | St. Dev | MEAN | St. Dev |
| ·24 | 11.1 | 7.7 | 21.7 | 5.6 | 3.7 | 3.3 | 3.0 | 3.0 | 276.7 | 182.8 |
| ·22 | 9.3 | 7.9 | 19.0 | 5.9 | 2.8 | 3.0 | 2.1 | 2.3 | 214.0 | 88.9 |
| ·20 | 8.5 | 6.1 | 18.4 | 6.9 | 2.6 | 3.2 | 1.7 | 2.2 | 208.7 | 96.0 |
| ·18 | 7.6 | 5.7 | 20.0 | 7.2 | 2.9 | 3.1 | 1.7 | 2.1 | 193.3 | 104.4 |
| ·16 | 7.3 | 5.7 | 17.8 | 8.1 | 3.2 | 3.1 | 1.6 | 2.0 | 203.3 | 135.0 |
| ·12 | 7.7 | 6.9 | 17.9 | 8.6 | 2.9 | 3.1 | 1.4 | 2.1 | 193.8 | 129 6 |
| ·1 | 14.3 | 10.7 | 22.8 | 7.9 | 2.9 | 3.1 | 3.4 | 3.4 | 482.1 | 342.7 |
| 2 | 10.1 | 7.2 | 19.0 | 6.9 | 3.3 | 3.5 | 2.4 | 2.3 | 244.0 | 166.1 |
| 4 | 18.4 | 11.5 | 21.7 | 9.0 | 3.2 | 4.2 | 3.0 | 2.5 | 254.5 | 170.2 |
| 6 | 21.9 | 14.7 | 19.5 | 8.7 | 2.5 | 3.2 | 4.2 | 2.6 | 222.0 | 102.9 |
| 8 | 29.0 | 15.1 | 20.6 | 7.7 | 2.5 | 3.5 | 5.1 | 2.3 | 222.9 | 117.7 |
| 12 | 28.4 | 13.8 | 17.5 | 7.6 | 2.6 | 3.7 | 5.8 | 2.1 | 186.2 | 104.0 |
| 24 | 27.8 | 12.1 | 22.6 | 7.3 | 3.0 | 3.5 | 6.2 | 3.3 | 318.3 | 177.0 |
| 36 | 26.3 | 11.6 | 21.3 | 8.2 | 4.5 | 3.8 | 6.6 | 2.4 | 207.5 | 134.3 |
| 48 | 27.5 | 13.7 | 21.8 | 8.4 | 2.7 | 3.3 | 5.9 | 3.1 | 263.4 | 168.1 |
| 60 | 27.0 | 12.3 | 20.7 | 5.6 | 3.4 | 4.4 | 5.7 | 1.6 | 200.2 | 63.6 |
| 72 | 25.2 | 14.3 | 20.5 | 8.2 | 3.8 | 3.4 | 5.8 | 3.3 | 310.5 | 298.7 |
| 84 | 25.4 | 13.9 | 16.5 | 8.6 | 2.5 | 2.9 | 5.1 | 2.4 | 164.2 | 81.4 |
| 95 | 23.2 | 11.3 | 21.5 | 7.6 | 2.2 | 3.1 | 5.9 | 2.3 | 285.6 | 165.2 |
| 96 | 16.4 | 9.8 | 18.7 | 7.2 | 2.2 | 3.0 | 4.0 | 3.0 | 222.2 | 142.2 |
| 100 | 14.2 | 9.7 | 18.8 | 10.0 | 3.3 | 4.8 | 3.4 | 2.4 | 188.4 | 104.2 |
| 108 | 7.8 | 5.6 | 17.5 | 7.6 | 2.7 | 3.3 | 1.0 | 1.8 | 158.4 | 83.6 |
| 120 | 11.5 | 7.4 | 20.6 | 6.2 | 3.0 | 3.2 | 2.7 | 2.6 | 296.6 | 184.0 |

2. ETMTDS

Serum concentrations of testosterone and estradiol increased soon after application and reached plateau levels within 12-24 hours. Mean (SD) peak uncorrected serum concentrations of T was 33.7 ng/dl (Range: 11-64 ng/dl) while $C_{max}$ for estradiol was 42.9 pg/ml (Range: 21-70 pg/ml). The primary metabolites of T and E2, DHT and estrone, had mean $C_{max}$ of 7.3 ng/dl and 36.8 pg/ml, respectively. The concentrations of T, E2, DHT, and E1 were all maintained in a narrow range over hours 24 - 96. As was the case for the TMTDS patch, DHEA serum concentrations maintained their characteristic circadian pattern (Range: 259 - 771) and appear to be unaffected by delivery of the exogenous sex hormones. Mean (SD) concentrations of each of the hormones measured during the ETMTDS wearing period are presented in Table 2 below.

TABLE 2

| TIME | TESTOSTERONE (ng / dl) | | ESTRONE (pg / ml) | | ESTRADIOL (pg / ml) | | DHT (ng / dl) | | DHEA (ng / dl) | |
|---|---|---|---|---|---|---|---|---|---|---|
| Hours | MEAN | St. Dev | MEAN | St. Dev | MEAN | St. Dev | MEAN | St. Dev | MEAN | St. Dev |
| ·24 | 12.9 | 9.8 | 21.6 | 6.6 | 3.3 | 2.9 | 2.6 | 2.9 | 294.2 | 179.2 |
| ·22 | 9.5 | 6 3 | 16.1 | 6.8 | 4.9 | 3.4 | 2.0 | 2.3 | 242.1 | 139.5 |
| ·20 | 9.7 | 7.3 | 17.1 | 7.1 | 2.1 | 3.1 | 1.4 | 2.0 | 242.1 | 164.2 |
| ·18 | 7.8 | 5.4 | 16.8 | 8.2 | 2.0 | 3.0 | 1.6 | 2.0 | 199.8 | 83.2 |
| ·16 | 8.8 | 6.7 | 18.6 | 7.3 | 4.1 | 3.1 | 1.4 | 1.8 | 209.7 | 114.6 |
| ·12 | 9.1 | 5.7 | 18.0 | 7.1 | 2.5 | 3.3 | 0.7 | 1.7 | 199.7 | 71.2 |
| ·1 | 15.1 | 11.7 | 20.8 | 7.0 | 2.1 | 3.8 | 2.2 | 2.9 | 408.2 | 278.5 |
| 2 | 10.4 | 6.9 | 18.9 | 5.3 | 3.7 | 3.3 | 1.6 | 2.0 | 208.5 | 81.6 |
| 4 | 15.0 | 10.0 | 20.0 | 6.0 | 8.4 | 7.2 | 2.0 | 2.4 | 226.1 | 115.4 |
| 6 | 19.2 | 11.6 | 20.7 | 6.4 | 15.8 | 11.5 | 3.1 | 2.1 | 228.9 | 142.2 |
| 8 | 26.6 | 17.5 | 22.8 | 7.4 | 24.8 | 17.8 | 4.0 | 3.0 | 227.8 | 104.2 |
| 12 | 23.0 | 12.9 | 22.9 | 6.5 | 23.5 | 9.5 | 4.4 | 3.1 | 201.3 | 120 8 |
| 24 | 29.3 | 11.0 | 31.9 | 6.7 | 32.8 | 13.5 | 6.7 | 2.8 | 349.4 | 183.1 |
| 36 | 27.1 | 12.7 | 31.9 | 6.8 | 37.2 | 16.4 | 5.0 | 3.7 | 178.8 | 78.2 |
| 48 | 28.1 | 13.2 | 34.3 | 7.7 | 32.6 | 15.2 | 5.8 | 2.5 | 294.9 | 164.0 |
| 60 | 25.8 | 12.7 | 32.9 | 9.2 | 36.0 | 16.0 | 4.7 | 1.6 | 171.8 | 67.2 |
| 72 | 24.1 | 10.1 | 33.3 | 7.0 | 31.1 | 9.7 | 5.5 | 2.8 | 282.3 | 141.6 |
| 84 | 24.6 | 12.3 | 31.2 | 5.8 | 34.6 | 13.6 | 4.2 | 2.6 | 175.1 | 68.2 |
| 96 | 24.8 | 11.8 | 31.8 | 7.4 | 29.0 | 11.4 | 5.2 | 2.9 | 265.5 | 120.3 |
| 98 | 18.1 | 10.2 | 29.9 | 7.0 | 20.4 | 5.8 | 4.4 | 2.3 | 247.5 | 133.6 |
| 100 | 12.1 | 8.6 | 30.1 | 7.3 | 15.0 | 4.3 | 2.4 | 2.7 | 198.5 | 75.7 |
| 108 | 6.6 | 6.1 | 25.2 | 7.1 | 7.6 | 3.5 | 1.4 | 2.3 | 168.4 | 6505.0 |
| 120 | 10.7 | 7.5 | 26.6 | 6.3 | 4.5 | 3.6 | 2.6 | 2.6 | 284.3 | 189.6 |

e. Conclusions

The TMTDS and ETMTDS were well tolerated by this population of postmenopausal, oophorectomized women. There were no significant skin or systemic adverse effects reported during the study. Both testosterone and estradiol were well absorbed from these formulations and resulting serum concentrations of both hormones approach the levels anticipated from in-vitro evaluation in a sustained manner over the proposed dosing interval.

The TMTDS raised circulating testosterone concentrations into the 25-30 ng/dl range, as did the ETMTDS, without excessive production of the major androgenic metabolite, DHT.

Delivery of estradiol from the ETMTDS provided mean incremental increases of circulating estradiol in the 25 to 30 pg/ml range.

**Claims**

1.   A skin patch for providing testosterone replacement therapy transdermally to a woman in need of such therapy, the patch comprising a laminate of:

(a) a backing layer; and
(b) a matrix layer comprising a solution of testosterone in a polymeric carrier being a pressure sensitive adhesive, the matrix layer containing a testosterone concentration sufficient for administering testosterone transdermally in an amount ranging from 50 to 500 μg/day, thereby causing blood levels of testosterone to be from 15 to 80 ng/dl.

2.   A skin patch according to claim 1 wherein the patch provides 100 to 300 μg testosterone/day.

3. A skin patch according to claim 1 wherein the matrix layer further includes an amount of estrogen equivalent to 25 to 200 μg/day of 17 β-estradiol, thereby resulting in an estrogenic activity level equivalent to a 17 β-estradiol blood level of 25 to 200 pg/ml.

4. A skin patch according to claim 3 wherein the estrogen is 17 β-estradiol.

5. The use of testosterone in the manufacture of a skin patch according to any preceding claim.

6. The use of estrogen in the manufacture of a skin patch according to claim 3 or 4.

**Patentansprüche**

1. Haulpflaster zum Bereitstellen einer transdermalen Testosteron-Ersetzungstherapie an eine Frau, die solch einer Therapie bedarf, wobei das Pflaster ein Laminat umfasst aus;

   (a) einer Verstärkungsschicht; und
   (b) einer Matrixschicht, umfassend eine Lösung von Testosteron in einem polymeren Trager, weicher ein druckempfindlicher Klebstoff ist, wobei die Matrixschicht eine ausreichende Testosteron-Konzentration zur transdermalen Verabreichung von Testosteron in einer Menge im Bereich von 50 bis 500 μg/Tag und **dadurch** Bewirkung von Blutspiegeln an Testosteron von 15 bis 80 ng/dl enthalt.

2. Hautpflaster nach Anspruch 1, wobei das Pflaster 100 bis 300 μg Testosteron/Tag liefert.

3. Hautpflaster nach Anspruch 1, wobei die Matrixschicht außerdem eine Menge an Östrogen enthält, die gleichwertig zu 25 bis 200 μg/Tag an 17β-Estradiol ist, wodurch eine östrogener Aktivitatsspiegel gleichwertig einem Blutspiegel an 17β-Estradiol von 25 bis 200 pg/ml erzielt wird

4. Hautpflaster nach Anpsruch 3, wobei das Östrogen 17β-Estradiol ist.

5. Verwendung von Testosteron in der Herstellung eines Haulpflasters nach jedem vorangehenden Anspruch.

6. Verwendung von Ostrogen in der Herstellung eines Hautpflasters nach Anspruch 3 oder 4.

**Revendications**

1. Timbre transdermique pour fournir une thérapie substitutive de testostérone par voie transdermique à une femme nécessitant une telle thérapie, le timbre comprenant un stratifié de

   (a) une couche support ; et
   (b) une couche matrice comprenant une solution de testostérone dans un véhicule polymère qui est un adhésif sensible à la pression, la couche matrice contenant une concentration de testostérone suffisante pour administrer de la testostérone par voie transdermique en une quantité allant de 50 à 500 μg/ jour, entraînant ainsi des taux sanguins de testostérone de 15 à 80 ng/dl.

2. Timbre transdermique selon la revendication 1, dans lequel le timbre fournit 100 à 300 μg de testostérone/jour.

3. Timbre transdermique selon la revendication 1 dans lequel la couche matrice comprend en outre une quantité d'oestrogène équivalente à 25 - 200 μg/jour de 17 β-oestradiol, résultant en un taux d'activité oestrogénique équivalent à un taux sanguin de 17 β-oestradiol de 25 à 200 pg/me.

4. Timbre transdermique selon la revendication 3 dans lequel l'oestrogène est le 17 β-oestradiol.

5. Utilisation de testostérone dans la fabrication d'un timbre transdermique selon l'une quelconque des revendications précédentes.

6. Utilisation d'oestrogène dans la fabrication d'un timbre transdermique selon la revendication 3 ou 4.

TS:ES CUMULATIVE FLUX RATIO

6 5 4 3 2 1 0

C7　CIN1605　CIN 1541　CIN 1573　CIN 1613　F42　CIN 1584　CIN 1630

SKIN

■ TS:ES ~ 3:1

● TS:ES ~ 4:1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5152997 A

- WO 9218107 A